# EUROPÄISCHE PATENTSCHRIFT

(11) **EP 2 754 404 B1**
(45) Veröffentlichungstag und Bekanntmachung des Hinweises auf die Patenterteilung: **19.04.2017**
(21) Anmeldenummer: 13151278.2
(22) Anmeldetag: 15.01.2013
(51) Int. Cl.: A61B 90/00, A61B 50/20

(54) **Augenoperationsgerät**
Ophthalmic surgery device
Appareil pour opérations oculaires

(43) Veröffentlichungstag der Anmeldung: 16.07.2014
(73) Patentinhaber: EOS GmbH, 52249 Eschweiler (DE)
(72) Erfinder: Lucke, Klaus, 28355 Bremen (DE)
(74) Vertreter: Röggla, Harald

(56) Entgegenhaltungen:
- DE-A1-102009 005 642
- US-A- 6 158 437
- US-A1- 2004 186 683
- US-A1- 2006 142 739
- US-A1- 2010 174 415
- US-A1- 2011 190 690

## Beschreibung

Die Erfindung betrifft ein Gerät zur Verwendung in der Chirurgie, an das ein Operationsutensil anschließbar ist, um eine Operation an einem Patienten durchzuführen, wobei Eingabemittel zum Steuern von Operationsparametern und Anzeigemittel zum Anzeigen der Operationsparameter vorgesehen sind.

Das Dokument EP 1 428 541 B1 offenbart so ein Gerät mit dem bei einer Katarakt-Operation eine neue Linse in das Auge eines Patienten eingesetzt werden kann. Bei der Operation wird vorerst von dem Operateur mit einem chirurgischen Handstück ein Schnitt an dem Auge gesetzt, über den die in kleine Stücke zerteilte alte Linse entfernt und anschließend die neue Linse in das Auge eingebracht wird. Um das Entfernen der kleinen Stücke der alten Linse zu ermöglichen und um zu verhindern, dass das von der alten Linse ursprünglich eingenommene Volumen im Auge bei der Operation kollabiert, muss von dem chirurgischen Handstück das Fluid mit einem Irrigationsdruck in das operierte Auge abgegeben werden. Ein zu hoher Irrigationsdruck würde das Auge bleibend schädigen und bei einem zu geringen Irrigationsdruck ist die Gefahr des Kollabierens des Auges gegeben, weshalb der Operateur den geeigneten Irrigationsdruck einstellen können muss. Dieser und weitere Operationsparameter müssen vom Operateur am Gerät eingestellt und während der Operation laufend angepasst werden.

Weiters wird es bei Operationen immer wichtiger jede Handlung des Operateurs oder der Operationsschwester zu dokumentieren, um einen hohen Qualitätsstandard bei Operationen zu gewährleisten und für mögliche Klagen ausreichend Beweismaterial zu haben. In diesem Zusammenhang sind Geräte aus anderen Fachbereichen bekannt, die ein Operationsprotokoll erstellen, in dem die jeweils eingestellten Operationsparameter während der Operation aufgezeichnet und gespeichert werden.

Bei dem bekannten Gerät hat sich als Nachteil ergeben, dass das Gerät und hierbei insbesondere die Eingabemittel des Geräts während der Operation stark verschmutzen und nur schwer wieder gereinigt werden können. Weiters hat sich bei dem bekannten Gerät als nachteilig erwiesen, dass das Operationsprotokoll in einzelnen Fällen nicht ausgereicht hat, um den Ablauf der Operation zuverlässig und vollständig nachzuvollziehen. Dokument US 2006/142739 A1 offenbart ein Gerät zur Verwendung in der Chirurgie, wobei Anzeigemittel zum Anzeigen der Operationsparameter vorgesehen sind, wobei das Gerät eine im Betrieb waagrechte Lichtmatrix und ein in einem Aufstellwinkel senkrecht nach oben verlaufendes Display aufweist, und wobei das Gerät Positionsdetektionsmittel aufweist, um auf der Lichtmatrix abgelegte kontaktlos kommunizierende Operationsutensilien bezüglich ihrer auf der Lichtmatrix zu detektieren. Der Erfindung liegt die Aufgabe zugrunde ein Gerät zur Verwendung in der Augenchirurgie zu schaffen, bei dem die vorstehend angeführten Nachteile vermieden werden. Erfindungsgemäß,wie in Anspruch 1 beansprucht, wird diese Aufgabestellung dadurch gelöst, dass das Gerät ein im Betrieb im Wesentlichen waagrechtes erstes Display und ein in einem Aufstellwinkel schräg nach oben verlaufendes zweites Display aufweist, wobei das Gerät Positionsdetektionsmittel aufweist, um auf dem ersten Display abgelegte kontaktlos kommunizierende Operationsutensilien bezüglich ihrer Position auf dem ersten Display zu detektieren. Hierdurch ist der Vorteil erhalten, dass das Gerät die auf dem ersten Display abgelegten Operationsutensilien detektiert, wodurch eine Vielzahl an Features ermöglicht sind, um das Operationsprotokoll zu verbessern und das Gerät besser zu reinigen. So hat es sich als vorteilhaft erwiesen am ersten Display die für die jeweilige Augenoperationsart benötigten Operationsutensilien naturgetreu dargestellt anzuzeigen und die Operation erst dann freizugeben, wenn entweder alle geforderten Operationsutensilien auf ihrem Platz am ersten Display liegen oder, wenn eine Abfrageinformation an den Operateur oder die Operationsschwester beantwortet wurde.

Besonders vorteilhaft ist es auch in dem Operationsprotokoll festzuhalten, wann welches Operationsutensil von dem ersten Display entfernt und wieder hingelegt wurde. Hierdurch kann nicht nur die Operation im Nachhinein anhand des Operationsprotokolls genau nachvollzogen werden, es können auch bestimmte Operationsparameter von der Gerätesteuerung automatisch eingestellt werden, wenn ein bestimmtes Operationsutensil von dem ersten Display des Geräts für die Operation genommen wurde. Auch die auf dem ersten Display und/oder auf dem zweiten Display dargestellten Anzeigeinformationen können je nach dem gerade verwendeten Operationsutensil für den Operateur geändert werden.

Durch die glatten Operflächen des ersten Displays und des zweiten Displays, die abgesehen von einem Fußschalter sämtliche Eingabemittel und Anzeigemittel des Geräts bilden, kann das Gerät besonders gut gereinigt werden. Dies ist bei einem in einem Operationssaal zu verwenden Gerät besonders wichtig und vorteilhaft.

Weitere vorteilhafte Ausgestaltungen des erfindungsgemäßen Geräts, wie in den abhängigen Ansprüche beansprucht, werden im Folgenden anhand der Figuren näher erläutert.
Figur 1 zeigt ein Gerät zur Verwendung in der Augenchirurgie.
Figur 2 zeigt ein Blockschaltbild der wesentlichen Funktionsblöcke des Geräts gemäß Figur 1.
Figur 3 zeigt ein Gerät zur Verwendung in der Chirurgie schematisiert von der Seite.

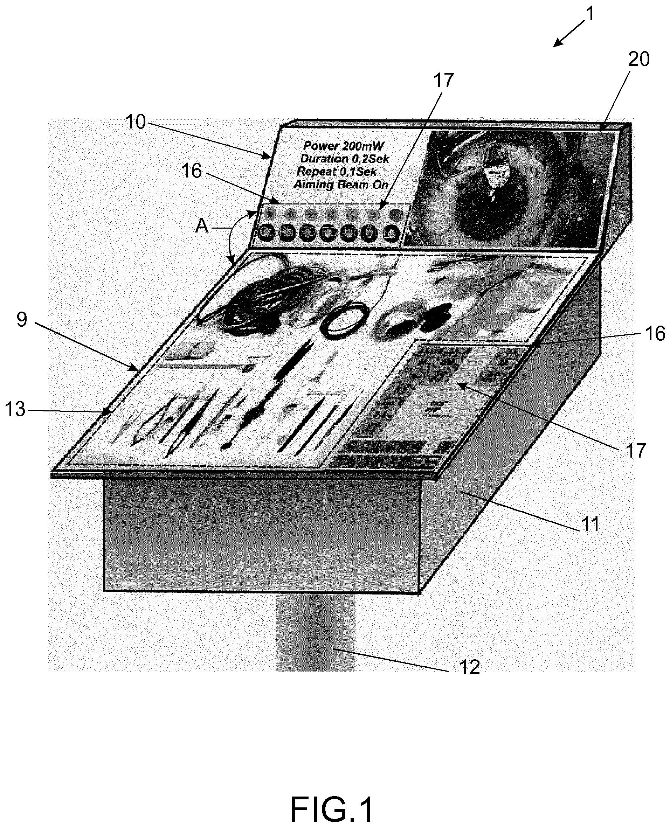

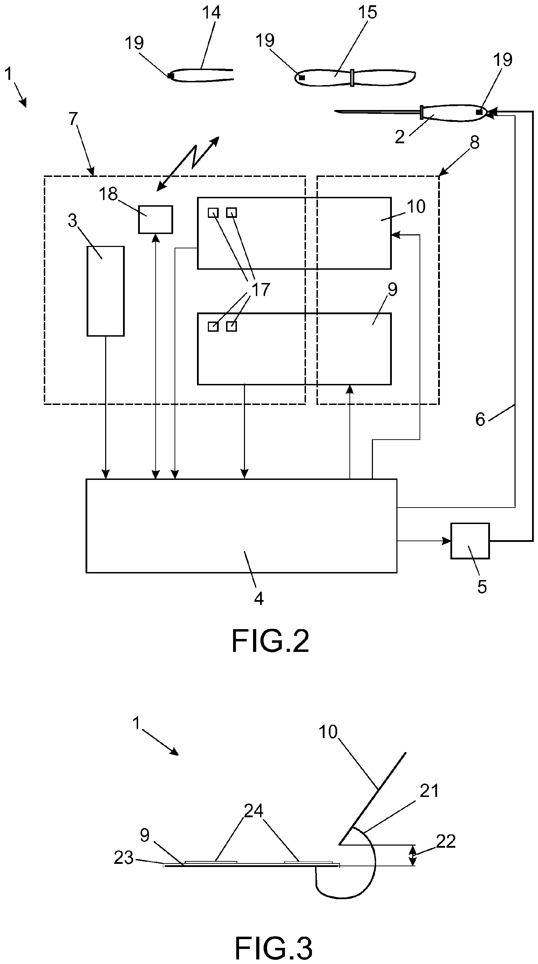

Figur 1 zeigt ein Gerät 1 zur Verwendung in der Augenchirurgie, an das ein in dem Blockschaltbild der Figur 2 dargestelltes chirurgisches Handstück 2 anschließbar ist. Mit dem Gerät 1 kann eine neue Linse in das Auge eines Patienten eingesetzt werden. Bei der Operation wird vorerst von dem Operateur ein Schnitt an dem Auge gesetzt und anschießend das Auge mit dem chirurgischen Handstück 2 operiert.

Das chirurgisches Handstück 2 erfüllt während der Operation drei Funktionen. An einer Irrigationsöffnung wird Fluid bzw. Spülflüssigkeit zum Spülen des Auges von dem chirurgischen Handstück 2 mit einem von dem Operateur mit einem Fußschalter 3 vorgegebenen Irrigationsdruck in das Auge abgegeben. Ein in dem chirurgischen Handstück 2 vorgesehenes mit einem Piezo angetriebenes Messer zerteilte die alte Linse in kleine Stücke, die zusammen mit der Spülflüssigkeit über eine Aspirationsöffnung des chirurgischen Handstücks 2 eingesaugt und in einer Kassette des Geräts 1 gesammelt werden.

Mit dem Fußschalter 3 kann der Operateur sowohl den momentan gewünschten Irrigationsdruck als auch den momentan gewünschten Unterdruck an der Aspirationsöffnung des chirurgischen Handstücks 2 zum Absaugen vorgeben. Das Gerät 1 weist weiters eine in Figur 2 dargestellte, durch einen Computer samt Computerprogramm des Geräts 1 realisierte Gerätesteuerung 4 auf, die sowohl den durch den Fußschalter 3 vorgegebenen Irrigationsdruck an der Irrigationsöffnung als auch den vorgegebenen Unterdruck an der Aspirationsöffnung steuert. Dies erfolgt durch Druckmittel 5, die durch einen in seiner Höhe verstellbaren Ständer der Spülflüssigkeit oder durch Druckbeaufschlagungsmittel realisiert sind. Die Gerätesteuerung 4 steuert über eine Steuerleitung 6 auch den Piezo des Messers im chirurgischen Handstück 2 an.

Das Gerät 1 weist nunmehr zusätzlich zu dem Fußschalter 3 weitere Eingabemittel 7, zum Steuern von Operationsparametern, und Anzeigemittel 8, zum Anzeigen der Operationsparameter und anderer für den Operateur interessanter Informationen, auf. Diese Eingabemittel 7 und Anzeigemittel 8 sind durch ein erstes im Wesentlichen waagrechtes Display 9 und ein vom Operateur aus gesehen dahinter liegendes in einem Aufstellwinkel A schräg nach oben verlaufendes zweites Display 10 gebildet. Das erste Display 9 liegt gemäß diesem Ausführungsbeispiel auf einem Gehäuse 11, das mittels eines Gerätefußes 12 auf dem Boden des Operationssaals steht.

Auf dem ersten Display 9 werden naturgetreue Darstellungen der einzelnen für die jeweilige Augenoperation zu verwendenden Operationsutensilien angezeigt. Je nach Art der Augenoperation werden unterschiedliche Operationsutensilien während der Operation benötigt, die vor der Operation von der Operationsschwester hergerichtet werden müssen. In Figur 1 sind in einem Anzeigebereich 13 eine Vielzahl solcher Operationsutensilien dargestellt, wobei in Figur 2 beispielhaft eine Pinzette 14, ein Messer 15 und das chirurgische Handstück 2 dargestellt sind.

Das erste Display 9 und das zweite Display 10 bilden, zusätzlich zu dem Fußschalter 3, auch die Eingabemittel 7, wofür in einem weiteren Anzeigebereich 16 Tasten 17 dargestellt sind, die als Touchscreen realisiert sind. Mit den Tasten 17 kann die jeweilige Augenoperationsart ausgewählt werden und es können weitere Eingaben, wie beispielsweise die Einstellung von Operationsparametern oder die Eingabe von Patientenidentifikationen, gemacht werden.

Das Gerät 1 weist nunmehr Positionsdetektionsmittel 18 auf, um auf dem ersten Display 9 abgelegte kontaktlos kommunizierende Operationsutensilien zu detektieren. Die Positionsdetektionsmittel 18 sind durch einen RFID-Reader im HF- oder UHF-Frequenzbereich sowie mehrere verteilt im ersten Display 9 beziehungsweise im Gehäuse 11 angeordnete Antennen gebildet. Die Position und Sendeleistung dieser Antennen wird hierbei so gewählt, dass die Position eines mit einem RFID-Label 19 (Aufkleber, der einen passiven und kontaktlos auslesbaren RFID-Tag enthält) versehenen Operationsutensils detektiert werden kann. So könnte zum Beispiel zyklisch nacheinander jeweils eine Antenne Sendeleistung lokal begrenzt abgeben, worauf die Seriennummer jener RFID-Labels 19 ausgelesen werden könnte, die sich in diesem lokal begrenzten Sendebereich auf dem ersten Display 9 befinden. Nach einem Durchlauf aller Antennen könnten die aus den RFID-Labels 10 ausgelesenen Seriennummern mit einer gespeicherten Tabelle von Seriennummern und zugeordneten Operationsutensilien verglichen werden, um diese zu indentifizieren. Je nach dem, von welcher Antenne die jeweilige Seriennummer mit welcher Empfangsleistung empfangen wurde, könnte die Position des jeweiligen Operationsutensils auf dem ersten Display 9 detektiert werden.

Auf diese Weise könnten die Positionsdetektionsmittel 18 detektieren, ob ein bestimmtes Operationsutensil tatsächlich dort liegt, wo es liegen sollte, welche vorgegebene Position durch die Anzeige der naturgetreuen Darstellung dieses Operationsutensils festgelegt wird. Die Operationsschwester könnte somit zur Vorbereitung der Operation sämtliche für die Operation vorgesehenen Operationsutensilien an den vorgegebenen Positionen auf das erste Display 9 auflegen und dann eine Taste Betätigen, um eine Operationsfreigabe zum Beginn der Operation zu erhalten. Die Gerätesteuerung 4 würde hierauf die Positionsdetektionsmittel 18 ansteuern um zu prüfen, ob sämtliche in dem Anzeigebereich 13 dargestellten Operationsutensilien auch tatsächlich physisch auf dem ersten Display 9 an der vorgegebenen Position liegen. Wenn eines oder mehrere dieser Operationsutensilien gar nicht auf dem ersten Display liegen oder an einer falschen Position auf dem ersten Display 9 liegen, dann würde die Gerätesteuerung 4 eine entsprechende Anzeigeinformation an dem ersten Display 9 oder an dem zweiten Display 10 darstellen. Hierauf könnte die Operationsschwester entweder ihren Fehler korrigieren oder bestätigen, dass in diesem speziellen Fall ein bestimmtes Operationsutensil nicht nötig ist oder an einer anderen Position auf dem ersten Display 9 liegen soll. Diese Information würde dann auch in dem Operationsprotokoll vermerkt werden. Die Gerätesteuerung 4 ist andererseits bei Vorhandensein aller Operationsutensilien auf dem ersten Display 9 zum Anzeigen einer Operationsfreigabeinformation an dem ersten Display 9 und/oder dem zweiten Display 10 ausgebildet.

Hierdurch ist der Vorteil erhalten, dass mit Sicherheit alle für die Operation nötigen Operationsutensilien am Begin der Operation an ihrer, durch die Anzeige der naturgetreuen Darstellung der Operationsutensilien, vorgegebenen Position auf dem ersten Display 9 liegen. Und wenn nicht alle Operationsutensilien an ihrem Platz liegen, dann ist im Operationsprotokoll eine Begründung dafür gemeinsam mit dem Namen der eingebenden Person vermerkt.

Während der Operation wird auch jeweils mit protokolliert, wann welches Operationsutensil vom Operateur oder der Operationsschwester vom ersten Display 9 genommen wurde.

Auch am Ende der Operation ist unmittelbar klar, ob sämtliche Operationsutensilien wieder auf dem ersten Display 9 liegen. Wenn ein Operationsutensil wegen starker Verschmutzung oder Defekt während der Operation nicht auf das erste Display 9 sondern unmittelbar entfernt werden soll, dann ermöglicht die Menüführung ein Ausloggen dieses Operationsutensils. Dieses Ausloggen wird in dem Operationsprotokoll wieder der Person zugeordnet, die diese Tätigkeit durchgeführt hat. Hierdurch können Operationen wesentlich sicherer und nachvollziehbarer gemacht werden.

Die Gerätesteuerung 4 steuert weiters die mit dem ersten Display 9 und/oder die mit dem zweiten Display 10 dargestellte Anzeigeinformation und/oder die Operationsparameter in Abhängigkeit von dem aktuell von dem ersten Display 9 entfernten Operationsutensil an. So ist es beispielsweise möglich, dass der Operateur das chirurgische Handstück 2 von dem ersten Display 9 nimmt, was von den Positionsdetektionsmittel 18 detektiert und an die Gerätesteuerung 4 gemeldet wird. Hierauf aktiviert die Gerätesteuerung 4 die Druckmittel 5 entsprechend Vorgabewerten für diese Augenoperationsart und der jeweiligen Stellung des Fußschalters 3. Die Gerätesteuerung 5 verändert somit Operationsparameter in Abhängigkeit von dem jeweils von dem ersten Display 9 abgenommenen Operationsutensil. Wenn der Operateur das chirurgische Handstück 2 wieder auf das erste Display 9 legt, dann deaktiviert die Gerätesteuerung 4 die Druckmittel 5. Gemäß einem anderen Beispiel wäre es möglich, dass eine spezielle Lampe zur Beleuchtung des Umfelds des Auges aktiviert wird, wenn der Operateur die Pinzette 14 vom ersten Display 9 nimmt, um einen Fremdkörper aus dem Auge des Patienten zu entfernen.

Wenn der Operateur vor, während oder nach der Operation zusätzliche Information nachschauen möchte, dann kann er bestimmte Tasten 17 an dem ersten Display 9 oder dem zweiten Display 10 betätigen, um diese Informationen am zweiten Display 10 angezeigt zu erhalten. So kann der Operateur beispielsweise in der Krankenakte des Patienten, von links nach recht, wie bei einem Buch, blättern oder Zusatzinformationen von einem Server oder aus dem Internet holen und sich am zweiten Display anzeigen lassen. Ebenso wäre es möglich, dass auf dem chirurgischen Handstück 2 eine Kamera montiert ist, um ein vergrößertes Bild des Auges währende der Operation "life" in einem Anzeigebereich 20 des zweiten Displays 10 anzuzeigen. Durch diesen einfachen Zugriff des Operateurs auf gespeicherte oder aktuell aufgezeichnete Informationen kann der Operateur wesentlich bessere Entscheidungen während der Operation treffen.

Dadurch, dass das Gerät 1 sehr kompakt ausgebildet und seine Eingabemittel 7, abgesehen von dem Fußschalter 3, nur durch das erste Display 9 und das zweite Display 10 gebildet sind, kann das Gerät 1 besonders gut hygienisch gereinigt werden. Auch die Abnutzung von Schaltern oder die Gefahr des Eindringens von Flüssigkeiten von den Operationsutensilien oder von den Handschuhen des Bedienpersonals in das Gehäuse 11 des Geräts 1 ist durch die Auswahl der Eingabemittel 7 vorteilhafterweise verhindert.

Das mit dem Gerät 1 aufgezeichnete Operationsprotokoll umfasst nicht nur die eingestellte Augenoperationsart, die zeitliche Abfolge aller während der Operation eingestellter oder veränderter Operationsparameter und die Patientendaten sondern auch die zeitliche Abfolge, wann welches Operationsutensil auf dem ersten Display 9 gelegen ist beziehungsweise wann welches Operationsutensil von dem ersten Display 9 entfernt wurde. Hierdurch kann eine Operation im Nachhinein lückenlos nachvollzogen und die Entscheidungen des Operateurs überprüft werden.

Vorteilhaft ist weiters, dass einige Operationsparameter für bestimmte Augenoperationsarten bereits voreingestellt in dem Gerät 1 gespeichert sind und durch Auswahl der Augenoperationsart automatisch für die nächste Operation eingestellt werden.

Figur 3 zeigt ein Gerät 1 zur Verwendung in der Chirurgie schematisiert von der Seite. Bei diesem Gerät 1 ist das zweite Display 10 an dem ersten Display 9 mittels eines Haltebügels 21 befestigt. Der Haltebügel 21 übernimmt hierbei die mechanische Befestigung und in dem Haltebügel 21 verlaufen die Anschlusskabel zur Versorgung des zweiten Displays 10. Zwischen dem ersten Display 9 und dem zweiten Display 10 ist ein Spalt 22 vorgesehen, der die Reinigung der Displays 9 und 10 erleichtert und das Auflegen einer Abdeckfolie 23 auf das erste Display 9 und gegebenenfalls auch auf das zweite Display 10 ermöglicht. Die Abdeckfolie 23 kann aus hygienischen Gründen nach jeder oder nach einigen Operationen ausgetauscht werden.

Besonders vorteilhaft ist allerdings, dass die Abdeckfolie 23 mehrere Rippen 24 aufweist. Die Rippen 24 sind im Wesentlichen Deckungsgleich zu den an dem ersten Display 9 angezeigten Operationsutensilien angeordnet und bilden Positionierungsmittel zum Positionieren der auf dem ersten Display 9 abgelegten Operationsutensilien. Hierdurch ist verhindert, dass die Operationsutensilien von dem ersten Display 9 unbeabsichtigt herunterrollen können.

Es kann erwähnt werden, dass das zweite Display 10, wie in den Figuren dargestellt, vom Operateur aus gesehen hinter dem ersten Display 9 angeordnet sein kann, aber nicht muss. So wäre es beispielsweise möglich, das zweite Display an einer oder beiden Seiten des ersten Displays 9 anzuordnen. Zusätzlich könnte dann auch noch das hinten angeordnete Display 9 vorgesehen sein. Der Aufstellwinkel A kann von einer nur geringen Neigung von beispielsweise 5 Grad bis zu einer Neigung von 90 Grad zum im Wesentlichen waagrechten ersten Display festgelegt sein.

Es kann erwähnt werden, dass "im Wesentlichen waagrecht" so zu verstehen ist, dass auf dem ersten Display 9 aufgelegte Operationsutensilien nicht vom ersten Display 9 rutschen sollen. Eine leichte Schrägstellung des ersten Displays 9 ist somit je nach Anwendungsfall möglich.

Es kann erwähnt werden, dass in dem zweiten Display 10, beispielsweise im Bereich des Anzeigebereichs 16, eine oder mehrere Buchsen vorgesehen sein können, um Operationsutensilien elektrisch mit dem Gerät 1 zu verbinden.

Bei den vorstehenden Ausführungsbeispielen wurde die Verwendung des Geräts in der Augenchirurgie (Katarakt-Operationen, Vitrectomy-Operationen, Hinterauge-Operationen) erläutert. Das Gerät kann aber durch Anpassung der Gerätesteuerung und der mit den Displays 9 und 10 angezeigten Informationen für die Durchführung anderer Operationen angepasst werden.

## Patentansprüche

1. Gerät (1) zur Verwendung in der Chirurgie, an das ein Operationsutensil anschließbar ist, um eine Operation an einem Patienten durchzuführen, wobei Eingabemittel (7) zum Steuern von Operationsparametern und Anzeigemittel (8) zum Anzeigen der Operationsparameter vorgesehen sind, **dadurch gekennzeichnet, dass**
das Gerät (1) ein im Betrieb im Wesentlichen waagrechtes erstes Display (9) und ein in einem Aufstellwinkel (A) schräg nach oben verlaufendes zweites Display (10) aufweist, wobei das Gerät (1) Positionsdetektionsmittel (18) aufweist, um auf dem ersten Display (9) abgelegte kontaktlos kommunizierende Operationsutensilien (2, 14, 15) bezüglich ihrer Position auf dem ersten Display (9) zu detektieren, und wobei das Gerät (1) zum Anzeigen von naturgetreuen Darstellungen der einzelnen für die jeweilige Augenoperation zu verwendenden Operationsutensilien (2, 14, 15) in einem Anzeigebereich (13) des ersten Displays (9) ausgebildet ist und, dass die Positionsdetektionsmittel (18) zum Melden des Vorhandenseins oder zum Melden des Fehlens einzelner oder aller der dargestellten Operationsutensilien (2, 14, 15) an eine Gerätesteuerung (4) ausgebildet sind.

2. Gerät (1) gemäß Anspruch 1, **dadurch gekennzeichnet, dass** das Gerät (1) zur Durchführung einer Augenoperation verwendbar ist und, dass das an das Gerät (1) anschließbare Operationsutensil durch ein chirurgisches Handstück (2) gebildet ist.

3. Gerät (1) gemäß einem der vorstehenden Ansprüche, **dadurch gekennzeichnet, dass** die Gerätesteuerung (4) bei Vorhandensein aller angezeigter Operationsutensilien (2, 14, 15) auf dem ersten Display (9) zum Anzeigen einer Operationsfreigabeinformation an dem ersten Display (9) und/oder dem zweiten Display (10) ausgebildet ist.

4. Gerät (1) gemäß einem der vorstehenden Ansprüche, **dadurch gekennzeichnet, dass** die Gerätesteuerung (4) beim Fehlen zumindest eines der angezeigten Operationsutensilien (2, 14, 15) auf dem ersten Display (9) zum Anzeigen einer Abfrageinformation an dem ersten Display (9) und/oder dem zweiten Display (10) ausgebildet ist.

5. Gerät (1) gemäß einem der vorstehenden Ansprüche, **dadurch gekennzeichnet, dass** die Gerätesteuerung (4) zum Aufzeichnen eines Operationsprotokolls ausgebildet ist, in dem zusätzlich zu den jeweils eingestellten Operationsparametern festgehalten wird, wann welches Operationsutensil (2, 14, 15) vom ersten Display (9) entfernt und auf das erste Display (9) wieder hingelegt wurde.

6. Gerät (1) gemäß einem der vorstehenden Ansprüche, **dadurch gekennzeichnet, dass** die Gerätesteuerung (4) die mit dem ersten Display (9) und/oder zweiten Display (10) dargestellte Anzeigeinformation und/oder die eingestellten Operationsparameter des Geräts (1) in Abhängigkeit von dem aktuell von dem ersten Display (9) entfernten Operationsutensil (2, 14, 15) steuert beziehungsweise ändert.

7. Gerät (1) gemäß einem der vorstehenden Ansprüche, **dadurch gekennzeichnet, dass** das ersten Display (9) und/oder das zweite Display (10) Eingabemittel (7) bildet und, dass durch Betätigung dieser Eingabemittel (7) in der mit dem ersten Display (9) und/oder mit dem zweiten Display (19) anzeigbaren Anzeigeinformationen, insbesondere nach links und/oder nach rechts, geblättert werden kann.

8. Gerät (1) gemäß einem der vorstehenden Ansprüche, **dadurch gekennzeichnet, dass** das Gerät (1) einen Fußschalter (3) zur Steuerung einzelner Operationsparameter aufweist und, das sämtliche weiteren Eingabemittel (17) und/oder sämtliche Anzeigemittel (8) des Geräts (1) mittels des ersten Displays (9) und/oder des zweiten Displays (10) realisiert sind.

9. Gerät (1) gemäß einem der vorstehenden Ansprüche, **dadurch gekennzeichnet, dass** mit den Eingabemitteln (7) zumindest zwei unterschiedliche Augenoperationsarten auswählbar sind, wobei die Gerätesteuerung (4), abhängig von der jeweiligen Auswahl der Augenoperationsart, zum Anzeigen unterschiedlicher voreingestellter Anzeigeinformationen und zum Einstellen der Operationsparameter auf vorgegebene Operationsparameter ausgebildet ist.

10. Gerät (1) gemäß einem der vorstehenden Ansprüche, **dadurch gekennzeichnet, dass** die Positionsdetektionsmittel (18) einen RFID-Reader aufweisen, der zum Auslesen von an den Operationsutensilien (2, 14, 15) vorgesehenen kontaktlos kommunizierenden RFID-Tags oder RFID-Lables (19) ausgebildet ist, um die Operationsutensilien (2, 14, 15) zu identifizieren und deren Position auf dem ersten Display (9) zu detektieren.

11. Gerät (1) gemäß einem der vorstehenden Ansprüche, **dadurch gekennzeichnet, dass** zwischen dem ersten Display (9) und dem zweiten Display (10) ein Spalt (22) vorgesehen ist.

12. Gerät (1) gemäß einem der vorstehenden Ansprüche, **dadurch gekennzeichnet, dass** auf dem ersten Display (9) ein im Wesentlichen durchsichtiges und insbesondere abnehmbares Positionierungsmittel (23) vorgesehen ist, das im Wesentlichen Deckungsgleich zu den an dem ersten Display (9) angezeigten Operationsutensilien zum Positionieren der auf dem ersten Display (9) abgelegten Operationsutensilien ausgebildet ist.

## Claims

1. A device (1) for use in surgery to which a surgical implement is connectable in order to conduct surgery on a patient, wherein input means (7) for controlling operating parameters and display means (8) for indicating the operating parameters are provided, **characterized in that**
the device (1) comprises a first display (9) which is essentially horizontal during operation and a second display (10) which runs obliquely upwards at an installation angle (A), wherein the device (1) exhibits position detection means (18) for detecting contactlessly communicating surgical implements (2, 14, 15) deposited on the first display (9) with regard to their positions on the first display (9), and wherein the device (1) is configured for indicating true-to-life depictions of the individual surgical implements (2, 14, 15) to be used for the respective eye surgery in a display area (13) of the first display (9) and that the position detection means (18) are configured for notifying the presence or for notifying the absence of some or all of the depicted surgical implements (2, 14, 15) to a device control (4).

2. A device (1) according to claim 1, **characterized in that** the device (1) is usable for the execution of eye surgery and that the surgical implement connectable to the device (1) is formed by a surgical handpiece (2).

3. A device (1) according to any of the preceding claims, **characterized in that**, in the presence of all depicted surgical implements (2, 14, 15) on the first display (9), the device control (4) is configured for indicating a clearance-for-surgery information on the first display (9) and/or the second display (10).

4. A device (1) according to any of the preceding claims, **characterized in that**, in the absence of at least one of the depicted surgical implements (2, 14, 15) on the first display (9), the device control (4) is configured for indicating a query information on the first display (9) and/or the second display (10).

5. A device (1) according to any of the preceding claims, **characterized in that** the device control (4) is configured for recording a surgical protocol in which, in addition to the operating parameters set in each case, it is recorded when which surgical implement (2, 14, 15) was removed from the first display (9) and was put back down on the first display (9).

6. A device (1) according to any of the preceding claims, **characterized in that** the device control (4) controls or changes, respectively, the display information indicated with the first display (9) and/or the second display (10) and/or the adjusted operating parameters of the device (1) as a function of the surgical implement (2, 14, 15) which currently has been removed from the first display (9).

7. A device (1) according to any of the preceding claims, **characterized in that** the first display (9) and/or the second display (10) constitute(s) input means (7) and that, by actuating said input means (7), it is possible to leaf through the display information indicatable with the first display (9) and/or the second display (19), in particular to the left and/or to the right.

8. A device (1) according to any of the preceding claims, **characterized in that** the device (1) comprises a foot switch (3) for controlling individual operating parameters and that all further input means (17) and/or all display means (8) of the device (1) are implemented by means of the first display (9) and/or the second display (10).

9. A device (1) according to any of the preceding claims, **characterized in that**, with the input means (7), at least two different types of eye surgery are selectable, wherein the device control (4) is configured for indicating various preset pieces of display information and for adjusting the operating parameters to predetermined operating parameters, depending on the respective choice of the type of eye surgery.

10. A device (1) according to any of the preceding claims, **characterized in that** the position detection means (18) comprise an RFID reader which is configured for reading out contactlessly communicating RFID tags or RFID labels (19) provided on the surgical implements (2, 14, 15) in order to identify the surgical implements (2, 14, 15) and to detect their positions on the first display (9).

11. A device (1) according to any of the preceding claims, **characterized in that** a gap (22) is provided between the first display (9) and the second display (10).

12. A device (1) according to any of the preceding claims, **characterized in that** an essentially transparent and in particular detachable positioning means (23) is provided on the first display (9), which positioning means is configured so as to be essentially congruent with the surgical implements indicated on the first display (9) for positioning the surgical implements deposited on the first display (9).

## Revendications

1. Appareil (1) pour utilisation en chirurgie, auquel un outil opératoire peut être raccordé pour réaliser une opération sur un patient, dans lequel il est prévu des moyens d'entrée (7) pour commander des paramètres opératoires et des moyens d'affichage (8) pour afficher les paramètres opératoires, **caractérisé en ce que**
l'appareil (1) présente un premier affichage (9) sensiblement horizontal en service et un second affichage (10) s'étendant en oblique vers le haut selon un angle de montage (A), dans lequel l'appareil (1) présente des moyens de détection de position (18) pour détecter des outils opératoires (2, 14, 15) communiquant sans contact stockés sur le premier affichage (9) concernant leur position sur le premier affichage (9) et dans lequel l'appareil (1) est conçu pour afficher des représentations fidèles des outils opératoires individuels (2, 14, 15) à utiliser pour l'opération oculaire respective dans une zone d'affichage (13) du premier affichage (9) et **en ce que** les moyens de détection de position (18) sont conçus pour indiquer la présence ou indiquer l'absence d'outils opératoires individuels ou de tous les outils opératoires représentés (2, 14, 15) sur une commande d'appareil (4).

2. Appareil (1) selon la revendication 1, **caractérisé en ce que** l'appareil (1) peut être utilisé pour réaliser une opération oculaire et l'outil opératoire raccordable à l'appareil (1) est formé par une poignée chirurgicale (2).

3. Appareil (1) selon l'une quelconque des revendications précédentes, **caractérisé en ce que** la commande d'appareil (4) est conçue, en présence de tous les outils opératoires affichés (2, 14, 15) sur le premier affichage (9), pour afficher une information d'autorisation d'opération sur le premier affichage (9) et/ou sur le second affichage (10).

4. Appareil (1) selon l'une quelconque des revendications précédentes, **caractérisé en ce que** la commande d'appareil (4) est conçue, en l'absence d'au moins l'un des outils opératoires affichés (2, 14, 15) sur le premier affichage (9), pour afficher une information d'interrogation sur le premier affichage (9) et/ou sur le second affichage (10).

5. Appareil (1) selon l'une quelconque des revendications précédentes, **caractérisé en ce que** la commande d'appareil (4) est conçue pour enregistrer un protocole d'opération, dans lequel, en plus des paramètres opératoires respectivement réglés, il est établi si et quel outil opératoire (2, 14, 15) a été retiré du premier affichage (9) et remis en place sur le premier affichage (9).

6. Appareil (1) selon l'une quelconque des revendications précédentes, **caractérisé en ce que** la commande d'appareil (4) commande ou modifie l'information d'affichage représentée avec le premier affichage (9) et/ou le second affichage (10) et/ou les paramètres opératoires réglés de l'appareil (1) en fonction de l'outil opératoire (2, 14, 15) retiré couramment du premier affichage (9).

7. Appareil (1) selon l'une quelconque des revendications précédentes, **caractérisé en ce que** le premier affichage (9) et/ou le second affichage (10) forme(nt) des moyens d'entrée (7) et **en ce que**, par commande de ces moyens d'entrée (7), on peut faire défiler les informations d'affichage affichables avec le premier affichage (9) et/ou le second affichage (19) en particulier vers la gauche et/ou vers la droite.

8. Appareil (1) selon l'une quelconque des revendications précédentes, **caractérisé en ce que** l'appareil (1) présente un commutateur à pédale (3) pour commander des paramètres opératoires individuels et tous les autres moyens d'entrée (17) et/ou tous les moyens d'affichage (8) de l'appareil (1) sont réalisés au moyen du premier affichage (9) et/ou du second affichage (10).

9. Appareil (1) selon l'une quelconque des revendications précédentes, **caractérisé en ce que**, avec les moyens d'entrée (7), on peut choisir au moins deux types d'opération oculaire différents, dans lequel la commande d'appareil (4) est conçue, en fonction du choix respectif du type d'opération oculaire, pour afficher différentes informations d'affichage préréglées et pour régler les paramètres opératoires sur des paramètres opératoires prédéterminés.

10. Appareil (1) selon l'une quelconque des revendications précédentes, **caractérisé en ce que** les moyens de détection de position (18) présentent un lecteur RFID, qui est conçu pour lire des étiquettes RFID ou RFID-labels (19) communiquant sans contact prévues pour lire sur les outils opératoires (2, 14, 15), afin d'identifier les outils opératoires (2, 14, 15) et détecter leur position sur le premier affichage (9).

11. Appareil (1) selon l'une quelconque des revendications précédentes, **caractérisé en ce qu'**il est prévu un intervalle (22) entre le premier affichage (9) et le second affichage (10).

12. Appareil (1) selon l'une quelconque des revendications précédentes, **caractérisé en ce qu'**il est prévu sur le premier affichage (9) un moyen de positionnement (23) sensiblement transparent et en particulier amovible, qui est conçu avec sensiblement la même couverture sur les outils opératoires affichés sur le premier affichage (9) pour positionner les outils opératoires stockés sur le premier affichage (9).
